# EUROPÄISCHE PATENTSCHRIFT

(11) **EP 0 526 754 B1**
(45) Veröffentlichungstag und Bekanntmachung des Hinweises auf die Patenterteilung: **28.02.1996**
(21) Anmeldenummer: 92111644.8
(22) Anmeldetag: 09.07.1992
(51) Int. Cl.: A61M 1/14, A61M 1/28, A61K 33/10, A61K 31/19

(54) **Wässrige Lösungen und deren Verwendung**
Aqueous solutions and their use
Solutions aqueuses et ses utilisation

(30) Priorität: 03.08.1991 DE 4125819
(43) Veröffentlichungstag der Anmeldung: 10.02.1993
(73) Patentinhaber: Zander, Rolf, Prof.Dr., D-55124 Mainz (DE)
(72) Erfinder: Zander, Rolf, Prof.Dr., D-55124 Mainz (DE)
(74) Vertreter: Weber, Dieter, Dr.

(56) Entgegenhaltungen:
- EP-A- 0 150 115
- EP-A- 0 313 546
- EP-A- 0 399 918

## Beschreibung

Bicarbonathaltige Dialysier-, Substitutions- oder Infusionsflüssigkeiten für die Hämo- oder Peritonealdialyse, Hämofiltration oder Infusion sind bekannt. Derartige bicarbonathaltige Flüssigkeiten ergeben verschiedene galenische Probleme, insbesondere wenn solche Flüssigkeiten nicht unmittelbar nach ihrer Herstellung verwendet, sondern in Lagerbehältern aufbewahrt werden sollen.

Bicarbonatlösungen sind nicht stabil, da immer die Gefahr besteht, daß aus einer Bicarbonatlösung CO₂ entweicht und sich damit die Zusammensetzung der Lösung verändert. Bestimmte Inhaltsstoffe in solchen Flüssigkeiten, insbesondere Glucose und Aminosäuren, können nur bei saurem pH-Wert im Bereich von 5,0 bis 5,5 sterilisiert bzw. gelagert werden, da sonst Denaturierung und/oder Braunfärbung eintritt. Dialyse- oder Infusionslösungen aber müssen für ihre Verwendbarkeit sterilisierbar sein. Schließlich muß man Dialysepatienten zur Kompensation von Calciumverlusten mit der Dialyselösung häufig Calciumionen zuführen. Diese aber dürfen nicht bei alkalischem pH-Wert mit Carbonationen, die aus Bicarbonat entstehen können, zusammengebracht werden, da sonst unlösliches Calciumcarbonat ausfallen würde.

Aus der EP-A-0 161 471 ist ein Zweikammerbehälter zur Lagerung und Bereitstellung einer bicarbonathaltigen Dialysier-, Substitutions- oder Infusionsflüssigkeit bekannt, der aus einem speziellen Polymerisat besteht, das die beiden Kammern flüssigkeits- und gasdicht voneinander trennt. Die eine Kammer enthält eine bicarbonatfreie Säurelösung und die andere eine bicarbonathaltige alkalische Lösung. Vor dem Gebrauch können die beiden Kammern zum Vermischen der Inhalte miteinander verbunden werden, und über ein Auslaßrohr kann die gemischte Lösung ihrer Verwendung zugeführt werden.

Der gleichzeitige Einsatz von Alkalicarbonat und Alkalibicarbonat ist in dieser Druckschrift nicht beschrieben. Vielmehr wird ausdrücklich darauf hingewiesen, daß eines der beiden Alkalisalze zu verwenden ist. Der für solche Flüssigkeiten vorgesehene Lagerbehälter ist aufwendig herzustellen, da gewährleistet sein muß, daß kein CO₂ entweicht, damit sich die Zusammensetzung der bicarbonathaltigen alkalischen Lösung bei der Lagerung nicht verändert.

Die in der EP-A-0 161 471 beschriebenen wäßrigen Lösungen haben keine physiologischen Werte des pH, der Bicarbonat-Konzentration und des CO₂-Partialdruckes. Das gleiche gilt für die aus der EP-A-0 277 868 beschriebenen Dialyselösungen. Die bisher insbesondere in der kontinuierlichen ambulanten Peritonealdialyse verwendeten Dialyselösungen haben auch keine physiologische Zusammensetzung, sondern besitzen aus Stabilitätsgründen stets einen sauren pH-Wert, wie im Bereich von 5,2 bis 5,5. Solche sauren Dialyselösungen können zu Schädigungen des Peritoneums, zu Reizungen des Abwehrsystems des Körpers und zu Schmerzen in der Bauchhöhle führen.

Aus der DE-A-3 514 346 sind Flüssigkeiten, insbesondere zum Kalibrieren von CO₂-Analysegeräten bekannt, die im Kontakt mit der atmosphärischen Luft ihren Gesamt-CO₂-Gehalt nicht verändern, und die bestimmte Konzentrationen an Alkalicarbonat und Alkalibicarbonat enthalten. Bei diesen Lösungen geht es aber ausschließlich darum, eine Flüssigkeit mit einem CO₂-Partialdruck zu bekommen, welcher dem der atmosphärischen Luft entspricht, während eine Einstellung physiologischer Werte des Säure-Basen-Status dieser Flüssigkeit nicht angestrebt wird.

Die EP-A-0 313 546 beschreibt ein Verfahren zum Kalibrieren eines Meßgerätes mittels zweier lagerstabiler, unmittelbar vor der Kalibrierung zu vereinigender Lösungen, von denen die eine bicarbonatfrei und sauer und die andere carbonat- und bicarbonathaltig und alkalisch ist. Bei Vereinigung der in der EP-A-0 313 546 offenbarten Lösungen bekommt man aber keine Lösung mit physiologischen Werten des pH, der Bicarbonat-Konzentration und des CO₂-Partialdruckes.

Die der Erfindung zugrundeliegende Aufgabe bestand daher darin, zwei getrennte sterilisierbare Lösungen zu bekommen, die an der Luft haltbar sind, ohne daß besondere Vorrichtungen zur Verhinderung einer Abdiffusion oder Eindiffusion von Kohlendioxid erforderlich sind, und bei ihrer Vereinigung eine als Dialysier-, Substitutions- oder Infusionslösung verwendbare, wäßrige Lösung mit physiologischen Werten des pH, der Bicarbonat-Konzentration und des CO₂-Partialdruckes sowie mit metabolisierbaren Anionen wenigstens einer organischen Säure ergeben.

Diese Aufgabe wird erfindungsgemäß mit zwei getrennten sterilisierbaren wäßrigen Lösungen gelöst, von denen die eine eine bicarbonatfreie saure Lösung und die andere eine Alkalibicarbonat und Alkalibicarbonat enthaltende alkalische Lösung ist und die dadurch gekennzeichnet sind, daß je Liter des Gesamtvolumens der beiden Lösungen zusammen die saure Lösung 7,3 mmol ± 3 % wenigstens einer metabolisierbaren organischen Säure und die alkalische Lösung 19,1 mmol ± 3 % Alkalibicarbonat und 6,1 mMol ± 3 % Alkalicarbonat enthält.

Solchermaßen zusammengesetzte wäßrige Lösungen nach der Erfindung sind an Luft haltbar, ohne daß sie in besonderen Behältnissen, die ein Abdiffundieren oder Eindiffundieren von CO₂ verhindern, untergebracht werden müssen. So braucht man als Vorratsbehälter weder Glasflaschen, die in vielen Fällen schwierig zu handhaben sind, noch speziell aufgebaute gasundurchlässige Behältnisse, wie sie beispielsweise in der EP-A-0 161 471 beschrieben sind. Dieser Vorteil ist beispielsweise besonders gravierend bei der ambulanten Peritonealdialyse, bei der es für den Patienten bequem und vorteilhaft ist, einen normalen Beutel für die Dialyseflüssigkeit verwenden zu können und von Glasbehältern unabhängig zu sein.

Die erfindungsgemäßen Lösungen sind sterilisierbar, auch wenn sie beispielsweise Glucose oder Aminosäuren enthalten, da die saure bicarbonatfreie Lösung einen die Denaturierung oder Braunfärbung verhindernden pH-Wert in der Größenordnung von 5,0 hat.

Die Vorarbeiten zu der vorliegenden Erfindung haben gezeigt, daß Dialysier-, Substitutions- oder Infusionslösungen dann besonders geeignet sind, wenn ihr pH-Wert, ihre Bicarbonat-Konzentration und ihr CO₂-Partialdruck den physiologischen Blutplasmawerten entspricht. Diese physiologischen Werte des Säure-Basen-Status liegen für den pH-Wert bei 7,40 ± 0,05, für die Bicarbonat-Konzentration bei 24 mmol/l und für den CO₂-Partialdruck bei 5332 Pa (40 mm Hg). Wenn man in einer künstlich zusammengestellten wäßrigen Lösung diese Werte bekommt, wird bei Verwendung solcher Lösungen als Dialysier-, Substitutions- oder Infusionslösung verhindert, daß bezüglich des Säure-Basen-Status eine Über- oder Unterdosierung erfolgt und damit eine Acidose oder Alkalose erzeugt wird, daß bezüglich der Einstellung der Atmung eines Patienten eine Hyperventilation oder eine Hypoventilation beobachtet wird und daß am Ort der Applikation bei Verwendung als Infusionslösung lokale Reaktionen an der Vene oder bei Verwendung als Peritonealdialyselösung lokale Reaktionen am Peritoneum erfolgen.

Die erfindungsgemäßen wäßrigen Lösungen ergeben quantitativ die genannten physiologischen Werte, so daß die aufgezeigten Vorteile bei Anwendung dieser wäßrigen Lösungen erzielt werden.

Weiterhin ermöglichen die erfindungsgemäßen wäßrigen Lösungen den Zusatz aller möglichen gewünschten Elektrolyte, wie von Calcium- und/oder Magnesiumionen, ohne daß die Gefahr einer Ausfällung von Calcium- oder Magnesiumcarbonat besteht, da diese Ionen der bicarbonatfreien sauren Lösung zugesetzt werden können.

Metabolisierbare Anionen organischer Säuren sind zur Therapie von Acidosen erwünscht. In den erfindungsgemäßen wäßrigen Lösungen stellen diese Anionen Langzeitpuffer dar, da ihre Wirkung erst in einer Zeit von Minuten bis Stunden nach Verabreichung je nach Umsetzung im Leberstoffwechsel und je nach der oder den verwendeten organischen Säuren auftritt. Diese Langzeitpufferwirkung ergänzt die Sofortpufferwirkung des Bicarbonats in physiologischer Konzentration.

Bei Vereinigung der beiden erfindungsgemäßen Einzellösungen unter Bildung der fertigen Lösung reagieren die metabolisierbaren organischen Säuren der sauren Einzellösung primär quantitativ mit den Carbonationen der alkalischen Einzellösung, da die Carbonationen einen höheren pK als die Bicarbonationen besitzen. Aus den Carbonationen werden dabei Bicarbonationen gebildet, die erst sekundär quantitativ mit dem verbleibenden Rest der metabolisierbaren organischen Säuren unter Bildung von CO₂ + H₂O reagieren. Diese Sekundärreaktion produziert einen CO₂-Partialdruck von 5332 Pa (40 mm Hg).

Vor allem das Alkalicarbonat, aber auch das Alkalibicarbonat werden in dem erfindungsgemäßen Lösungssystem dazu benutzt, die Alkalisalze der zugesetzten metabolisierbaren organischen Säuren zu bilden, was für die Therapie entscheidend ist. Würde nämlich dem Organismus die organische Säure selbst, wie z. B. Milchsäure, zugeführt, so dissoziiert diese vollständig bei physiologischem pH von 7,40 und führt dementsprechend zu einer Acidose. Wird sie im Stoffwechsel abgebaut, wie in der Leber, so entstehen CO₂ + H₂O, also neutrale Endprodukte. Bei Milchsäureinfusion z. B. geht der pH-Wert sofort nach unten (Acidose) und normalisiert sich danach innerhalb von etwa 2 h.

Wird dagegen das Alkalisalz, wie Natriumlactat, dem Organismus zugeführt, so kommt es in Minuten bis Stunden entsprechend der Verstoffwechselung des Säuresalzes in der Leber zu einer Alkalisierung des Organismus, da das Alkalisalz pro Mol des Salzes 1 mol H⁺ in den Stoffwechsel mitnimmt. Bei einer Lactatinfusion z. B. bleibt der pH-Wert zunächst neutral und geht dann beispielsweise innerhalb von 2 h in den alkalischen Bereich.

Wie schnell ein metabolisierbares Anion zu einer Alkalisierung des Organismus führt, hängt von der Stoffwechsellage der Leber, von der Art des zugeführten Anions und von der Konzentration des Anions ab.

Die metabolisierbaren organischen Anionen werden Infusionslösungen zugesetzt, um prophylaktisch eine Alkalisierung des Organismus einzuleiten. Auch in Dialyselösungen werden metabolisierbare organische Anionen verwendet, um die Acidose des Dialysepatienten, die während der Dialyse noch zunimmt, zu kompensieren.

Aus galenischen Gründen enthält bis zum heutigen Tag keine Infusionslösung Bicarbonat, was zwangsläufig zu einer sogenannten Verdünnungsacidose führen muß, weil der Organismus pro Liter Lösung 24 mmol Bicarbonat aus seiner Reserve zur Verfügung stellen muß. Diese Verdünnungsacidose läßt sich in vitro wie auch in vivo nachweisen.

Die erfindungsgemäßen Lösungen bieten den weiteren Vorteil, daß bezüglich der metabolisierbaren Anionen, deren Alkalisierungseffekt erwünscht ist, keine Fehldosierung möglich ist, die ihrerseits zu einer iatrogenen Alkalose führen kann, welche möglicherweise eine Gefahr für den Patienten darstellt, da er diese Alkalose durch Hypoventilation kompensieren muß, letztere aber dadurch begrenzt ist, daß die Reduzierung der Atmung eine Hypoxie (Sauerstoffmangel) des Patienten erzeugen kann.

Die erfindungsgemäßen Lösungen enthalten somit den Sofortpuffer Bicarbonat in physiologischer Konzentration zusammen mit dem Langzeitpuffer metabolisierbares Anion in der gewünschten Konzentration. Bevorzugt in den wäßrigen Lösungen nach der Erfindung verwendbare metabolisierbare Säuren sind Brenztraubensäure, Milchsäure, Oxalsäure, Fumarsäure, Essigsäure, Äpfelsäure, Bernsteinsäure, Malonsäure und Maleinsäure.

Weiterhin ist es bevorzugt, die Abweichungen von 7,3 mmol der metabolisierbaren Säuren, 19,1 mMol des Alkalibicarbonats und 6,1 mmol des Alkalicarbonats je Liter der fertigen Lösung auf nur ±1 % einzustellen, um einen pH-Wert von 7,40 ± 0,05 zu erreichen und Abweichungen des CO₂-Partialdruckes von ±533 Pa (±4 mm Hg) einzuhalten.

Die oben angegebenen Millimolmengen für die einzelnen Komponenten beziehen sich auf das Volumen der aus den beiden Einzellösungen durch Vermischen erhaltenen fertigen Lösung, so daß die Volumina der sauren und alkalischen Einzellösungen beliebig variiert werden können, sofern nur deren Konzentrationen entsprechend der obigen Lehre eingestellt werden. Wenn man beispielsweise die saure und alkalische Einzellösung jeweils auf 1 l einstellt, so daß die fertige Lösung ein Volumen von 2 l hat, so braucht man in der sauren Lösung 14,6 mmol/l der metabolisierbaren organischen Säure und in der alkalischen Lösung 38,2 mmol/l Alkalibicarbonat und 12,2 mmol/l Alkalicarbonat. Bei Veränderung der Volumenverhältnisse der beiden Einzellösungen müssen die Konzentrationen entsprechend umgerechnet werden.

Wenn in Verbindung mit den Patentansprüchen und der Beschreibung von jeweils einer sauren und alkalischen Einzellösung die Rede ist, so soll dies einschließen, daß selbstverständlich das Gesamtvolumen der sauren und alkalischen Einzellösung jeweils unterteilt werden kann, was allerdings gewöhnlich keinen zusätzlichen Vorteil erbringt. Diese Teilmengen der alkalischen oder sauren Einzellösung können hinsichtlich der Zusammensetzung gleich oder verschieden sein, sofern nur in allen Teilmengen der Einzellösungen zusammen die oben angegebenen Millimole der aufgeführten Komponenten enthalten sind.

Zweckmäßig ist es weiterhin, daß die saure Einzellösung zusätzlich Calcium- und Magnesiumionen enthält. Wie bereits erwähnt, kann sie auch zusätzlich noch andere Stoffe, wie Glucose und/oder Aminosäuren enthalten.

Die wäßrigen Lösungen nach der Erfindung beseitigen vollständig die bisherige Problematik von Bicarbonatinfusionen. Bei Verabreichung von Bicarbonat zur Therapie einer Acidose muß CO₂ + H₂O entstehen. Die Verabreichung von relativ hohen Konzentrationen an Bicarbonat führt daher immer zu einer für den Patienten unangenehmen Hyperventilation, da das zwangsläufig gebildete CO₂ einen Atemreiz darstellt, der die Hyperventilation auslöst, um das zusätzlich gebildete CO₂ abzuatmen. Da die erfindungsgemäßen Infusionslösungen eine Bicarbonat-Konzentration von 24 mmol/l besitzen, wird eine Über- oder Fehldosierung von Bicarbonat in einer Infusionslösung automatisch ausgeschlossen.

Die saure Einzellösung kann zusätzlich zu der oder den metabolisierbaren organischen Säuren auch noch Salze dieser oder anderer metabolisierbarer organischer Säuren enthalten, um die erwünschte Konzentration an metabolisierbaren Anionen zu bekommen.

Die nachfolgenden Tabellen I bis III zeigen Beispiele für die Zusammensetzung der sauren Einzellösung in Verbindung mit der daraus resultierenden Basenkonzentration in der fertigen vereinigten Lösung nach Mischung 1 + 1 mit der alkalischen Lösung, wobei alle Angaben auf 37 °C bezogen sind und die Konzentrationen in Millimol/Liter angegeben sind.

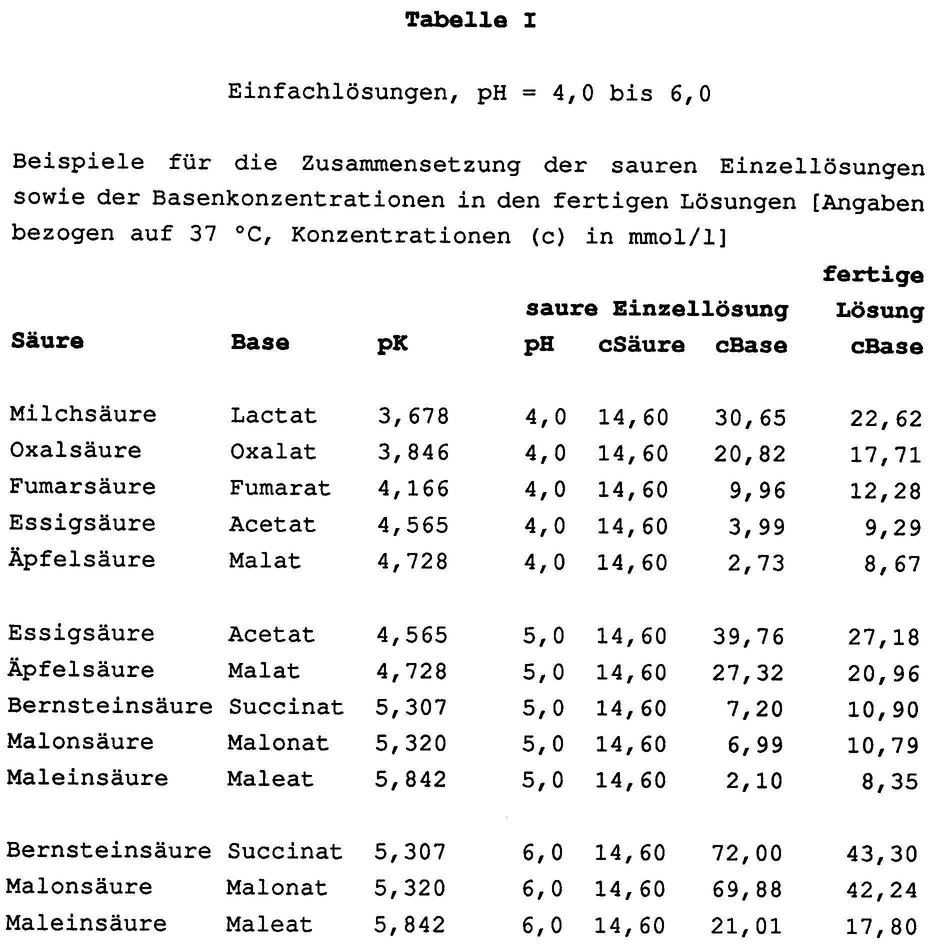

Die pK-Werte sind Meßwerte bei 37 °C und einer Ionenstärke von 160 mmol/l.

### Ergebnisse:

1. Je nach pH-Vorgabe der sauren Lösung, die aus galenischen Gründen erfolgt, kommt nur eine begrenzte Zahl von organischen Säuren in Frage.
2. Soll die Konzentration der Base der organischen Säure (metabolisierbares Anion) in einem therapeutischen Bereich zwischen etwa 10 und 50 mmol/l, bezogen auf die fertige Lösung, liegen, so kommt nur ein begrenzter pH-Bereich für die saure Einzellösung in Frage.

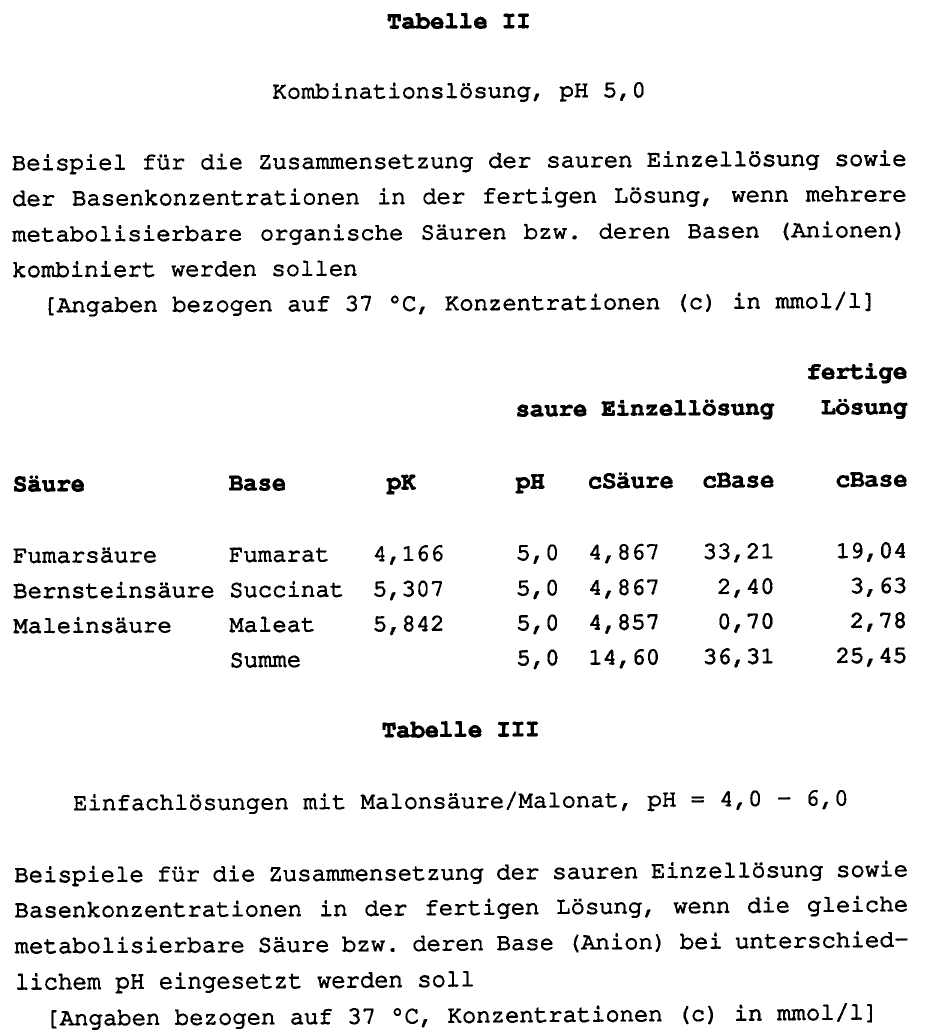

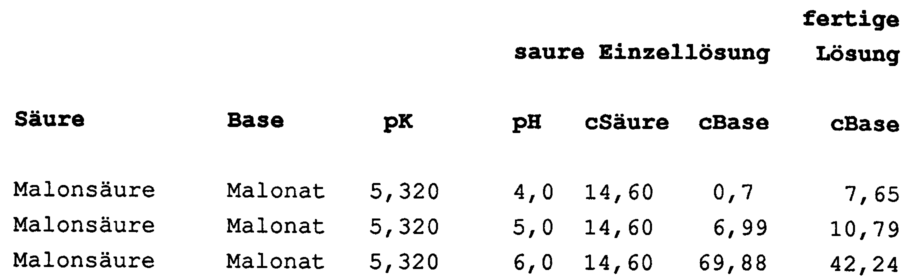

### Meßergebnisse

Messung des pH-Wertes (Mittelwerte von 10 Messungen, 37 °C) vor und nach Mischung der alkalischen Einzellösung (HCO₃⁻/CO₃⁻⁻) mit der sauren Einzellösung (Na⁺, K⁺, Ca⁺⁺, organische Säure, organische Base, Cl⁻) im Verhältnis 1 + 1 mit dem Ziel, daß die fertige Lösung pH = 7,40 ± 0,05, cHCO₃⁻ = 24,0 mmol/l, cNa⁺ = 140 mmol/l, cK⁺ = 4,0 mmol/l, cCa⁺⁺ = 2,5 mmol/l beträgt (je nach Konzentration der organischen Säure/Base muß die NaCl-Konzentration der sauren Einzellösung entsprechend eingestellt werden).

| | alkalische Einzellösung | saure Einzellösung | fertige Lösung |
|---|---|---|---|
| | | | |

| Lösungen gemäß Tabelle I mit einem pH von 5,0: | | | |
|---|---|---|---|
| Essigsäure/Acetat | 9,38 | 5,07 | 7,407 |
| Äpfelsäure/Malat | | 4,93 | 7,395 |
| Bernsteinsäure/Succinat | | 5,06 | 7,393 |
| Maleinsäure/Maleat | | 4,92 | 7,409 |

| Kombinationslösung gemäß Tabelle II: | | | |
|---|---|---|---|
| Fumarsäure/Fumarat | | | |
| Bernsteinsäure/Succinat | | | |
| Maleinsäure/Maleat | 9,39 | 4,99 | 7,401 |

| Lösungen gemäß Tabelle III: | | | |
|---|---|---|---|
| Malonsäure/Malonat | 9,39 | 4,16 | 7,412 |
| Malonsäure/Malonat | | 4,95 | 7,416 |
| Malonsäure/Malonat | | 5,94 | *7,519 |

| | | | |
|---|---|---|---|
| * Anmerkung | | | |

Da die Malonsäure einen relativ hohen pK-Wert hat (5,32) entwickelt sie bei pH = 7,40 dann eine minimale Pufferwirkung, wenn sie in hoher Konzentration (hier 42,24 mmol/l) vorliegt: CO₂(H₂CO₃) wird in Spuren gepuffert. Deshalb steigt der pH-Wert minimal an (der pCO₂ fällt).

Die Erfindung betrifft auch die Verwendung der oben beschriebenen wäßrigen Lösungen, insbesondere als Dialysier-, Substitutions- oder Infusionslösungen.

### Beispiel

In dem folgenden Ausführungsbeispiel (Angaben bezogen auf 37 °C) wurde die alkalische Einzellösung und die saure Einzellösung jeweils auf 1 l eingestellt. Die alkalische Einzellösung enthielt 38,2 mmol/l Natriumbicarbonat und 12,2 mmol/l Natriumcarbonat. Der pH-Wert dieser Lösung lag bei 9,4.

Die saure Einzellösung enthielt 14,6 mmol/l Essigsäure und 39,8 mmol/l Natriumacetat und besaß einen pH-Wert von 5,0.

Die beiden Lösungen waren völlig lagerbeständig ohne Verwendung spezieller Behältnisse, die das Eindiffundieren oder Ausdiffundieren von CO₂ verhindern. Ihre Zusammensetzung blieb über lange Zeiträume stabil.

Bei der Vereinigung der beiden Einzellösungen bekam man 2 l fertige Lösung mit einem CO₂-Partialdruck von 5332 Pa (40 mm Hg), einem pH-Wert von 7,40, einer Bicarbonatkonzentration von 24,0 mmol/l und einer Acetatkonzentration von 27,2 mmol/l. Diese Lösung kann mit Vorteil als Dialysier-, Substitutions- oder Infusionslösung verwendet werden.

## Patentansprüche

1. Zwei getrennte sterilisierbare wäßrige Lösungen, die bei der Vereinigung miteinander eine wäßrige Lösung mit im wesentlichen physiologischen Werten des pH, der Bicarbonat-Konzentration und des CO₂-Partialdruckes ergeben und von denen die eine eine bicarbonatfreie saure Lösung und die andere eine Alkalibicarbonat und Alkalicarbonat enthaltende alkalische Lösung ist, **dadurch gekennzeichnet**, daß je Liter des Gesamtvolumens der beiden Lösungen zusammen die saure Lösung 7,3 mmol ± 3 % wenigstens einer metabolisierbaren organischen Säure und die alkalische Lösung 19,1 mmol ± 3 % Alkalibicarbonat und 6,1 mmol ± 3 % Alkalicarbonat enthält.

2. Lösungen nach Anspruch 1, **dadurch gekennzeichnet**, daß je Liter des Gesamtvolumens der beiden Lösungen zusammen die saure Lösung 7,3 mmol ± 1 % der metabolisierbaren organischen Säuren und die alkalische Lösung 19,1 mmol ± 1 % Alkalibicarbonat und 6,1 mmol ± 1 % Alkalicarbonat enthält.

3. Lösungen nach Anspruch 1 oder 2, **dadurch gekennzeichnet**, daß die saure Lösung zusätzlich wenigstens ein Alkalisalz wenigstens einer metabolisierbaren organischen Säure enthält.

4. Lösungen nach Anspruch 3, **dadurch gekennzeichnet**, daß die saure Lösung Natriumsalze der metabolisierbaren organischen Säuren enthält.

5. Lösungen nach einem der Ansprüche 1 bis 4, **dadurch gekennzeichnet**, daß die alkalische Lösung als Alkalibicarbonat Natriumbicarbonat und als Alkalicarbonat Natriumcarbonat enthält.

6. Lösungen nach einem der Ansprüche 1 bis 5, **dadurch gekennzeichnet**, daß die saure Lösung zusätzlich Calcium- und/oder Magnesiumionen enthält.

7. Lösungen nach einem der Ansprüche 1 bis 6, **dadurch gekennzeichnet**, daß die saure Lösung als metabolisierbare organische Säuren Brenztraubensäure, Milchsäure, Oxalsäure, Fumarsäure, Essigsäure, Apfelsäure, Bernsteinsäure, Malonsäure und/oder Maleinsäure enthält.

8. Verwendung wäßriger Lösungen nach einem der Ansprüche 1 bis 7 zur Herstellung einer Dialysier-, Substitutions- oder Infusionslösung.

## Claims

1. Two separate sterilisable aqueous solutions which upon being brought together produce an aqueous solution with substantially physiological values in respect of the pH, biocarbonate concentration and CO₂-partial pressure and of which one is a bicarbonate-free acid solution and the other is an alkaline solution containing alkali metal bicarbonate and alkali metal carbonate, characterised in that per litre of the overall volume of the two solutions together the acid solution contains 7.3 mmol ± 3 % of at least one metabolisable organic acid and the alkaline solution contains 19.1 mmol ± 3 % of alkali metal bicarbonate and 6.1 mmol ± 3 % of alkali metal carbonate.

2. Solutions according to claim 1 characterised in that per litre of the total volume of the two solutions together the acid solution contains 7.3 mmol ± 1 % of the metabolisable organic acids and the alkaline solution contains 19.1 mmol ± 1 % of alkali metal bicarbonate and 6.1 mmol ± 1 % of alkali metal carbonate.

3. Solutions according to claim 1 or claim 2 characterised in that the acid solution additionally contains at least one alkali metal salt of at least one metabolisable organic acid.

4. Solutions according to claim 3 characterised in that the acid solution contains sodium salts of the metabolisable organic acids.

5. Solutions according to one of claims 1 to 4 characterised in that the alkaline solution contains sodium bicarbonate as the alkali metal bicarbonate and sodium carbonate as the alkali metal carbonate.

6. Solutions according to one of claims 1 to 5 characterised in that the acid solution additionally contains calcium and/or magnesium ions.

7. Solutions according to one of claims 1 to 6 characterised in that as metabolisable organic acids the acid solution contains pyruvic acid, lactic acid, oxalic acid, fumaric acid, acetic acid, malic acid, succinic acid, malonic acid and/or maleic acid.

8. Use of aqueous solutions according to one of claims 1 to 7 for the production of a dialysing, substitution or infusion solution.

## Revendications

1. Deux solutions aqueuses séparées stérilisables et qui, quand on les réunit ensemble, donnent une solution aqueuse, présentant des valeurs essentiellement physiologiques du pH, de la concentration en bicarbonate et de la pression partielle en CO₂ et dont l'une est une solution acide dépourvue de bicarbonate et l'autre est une solution alcaline contenant du bicarbonate alcalin et du carbonate alcalin, caractérisées en ce que, par litre du volume total des deux solutions prises ensemble, la solution acide contient 7,3 mmoles ± 3 % d'au moins un acide organique métabolisable et la solution alcaline contient 19,1 mmoles ± 3 % de bicarbonate alcalin et 6,1 mmoles ±3% de carbonate alcalin.

2. Solutions selon la revendication 1, caractérisées en ce que chaque litre du volume total des deux solutions prises ensemble contient, pour la solution acide, 7,3 mmoles ± 1 % des acides organiques métabolisables, et pour la solution alcaline, 19,1 mmoles ± 1 % de bicarbonate alcalin et 6,1 mmoles ± 1 % de carbonate alcalin.

3. Solutions selon la revendication 1 ou 2, caractérisées en ce que la solution acide contient en outre au moins un sel alcalin d'au moins un acide organique métabolisable.

4. Solutions selon la revendication 3, caractérisées en ce que la solution acide contient des sels de sodium des acides organiques métabolisables.

5. Solutions selon l'une des revendications 1 à 4, caractérisées en ce que la solution alcaline contient, comme bicarbonate alcalin, du bicarbonate de sodium et, comme carbonate alcalin, du carbonate de sodium.

6. Solutions selon l'une des revendications 1 à 5, caractérisées en ce que la solution acide contient en outre, des ions calcium et/ou magnésium.

7. Solutions selon l'une des revendications 1 à 6, caractérisées en ce que la solution acide contient, comme acides organiques métabolisables, de l'acide pyruvique, de l'acide lactique, de l'acide oxalique, de l'acide fumarique, de l'acide acétique, de l'acide malique, de l'acide succinique, de l'acide malonique, et/ou de l'acide maléïque.

8. Utilisation de solutions aqueuses selon l'une des revendications 1 à 7 pour la préparation d'une solution pour dialyse, pour substitution ou remplacement ou pour infusion.
